# EUROPEAN PATENT APPLICATION

(11) **EP 3 295 968 A1**
(43) Date of publication of application: **21.03.2018**
(21) Application number: 16189159.3
(22) Date of filing: 16.09.2016
(51) Int. Cl.: A61L 27/26, A61L 27/52, A61L 15/28

(54) **COMBINATION OF COLLAGEN AND CARBOXYMETHYL CELLULOSE AND USE THEREOF AS A DERMAL FILLER**

(71) Applicant: Euroresearch S.r.l., 20129 Milano (IT)
(72) Inventor: SCALESCIANI, Juan Francisco, I-20129 Milano MI (IT)
(74) Representative: Gislon, Gabriele

(57) **Abstract**

Subject-matter of the invention is a combination of collagen and carboxymethyl cellulose (CMC), the injectable compositions comprising it and their use as dermal filler. Another subject-matter of the invention are means for the administration of said compositions, more particularly pre-filled syringes.

## Description

### Technical Field

Injectable compositions comprising substances that produce a dermal filling effect, thus providing an increase of the subcutaneous volume and/or reducing skin defects, have long been known.

By way of example, several filling agents are known usually called "fillers", which are used to improve the aesthetic aspect of wrinkles and other signs of aging, as well as to make less evident the skin imperfections, such as scars and other results of epithelial lesions.

Collagen is considered a substance with regenerative and reconstructive actions on the tissues and therefore is one of the substances used for these purposes. Collagen is a very abundant scleroprotein in higher vertebrates because it is one of the main components of the skin and connective tissue and, in addition, it is a basic constituent of tendons and bones.

Several types of collagen are known; type I collagen is the most abundantly present in vertebrates. The role of collagen in wound healing and reepithelialization is known and, for this reason, this protein is usually employed in the treatment of ulcers, bums and similar diseases as well.

Carboxymethyl cellulose (CMC) is a cellulose derivative wherein part of the hydroxy groups (OH) is replaced with carboxymethyl groups (-CH₂-COOH).

The addition of these carboxymethyl groups increases the steric hindrance of the molecule and, moreover, the addition of these groups makes the molecule more stable, i.e. less degradable.

CMC has been used, usually as sodium salt, CMCNa, as dermal filler with decent success because it is a low- or non-allergenic substance and low cost. Fillers and filler combinations known until now involve the disadvantage of requiring frequent injections, because they are very quickly reabsorbed and/or metabolized by the organism. In view of this problem, CMC used as filler is usually cross-linked to improve its stability; however the use of cross-linking agents, such as for example 1,4-butanediol diglycidyl ether, requires the purification of reacted CMC in order to remove the unreacted cross-linking agent and avoid its administration together with the CMC itself. Purification processes are expensive and cannot completely remove the cross-linking agent from the network formed by the reacted CMC.

There is therefore the need of finding new combinations of substances that allow to obtain alternative fillers, which are easy to be injected also through needles with very small diameters, e.g. of the order of 0.2-0.13 mm, and which provide satisfying and long-lasting aesthetic results.

### Objects of the Invention

It is an object of the invention is to provide a new combination of substances to be administered as dermal filler which have typical filling effect and, at the same time, regenerative nature.

It is another object of the invention to provide injectable compositions which are easy to be injected and provide long-lasting results.

It is another object of the invention to provide injectable compositions comprising the combination of the invention.

It is a further object of the invention to provide means for the administration of the compositions of the invention, which are effective, safe and ready to use.

### Description of the Invention

Therefore subject-matter of the invention, according to one of the aspects thereof, is a combination of collagen and carboxymethyl cellulose (CMC), for use as dermal filler and tissue regenerating agent.

More particularly, subject-matter of the invention is a fixed combination of collagen and CMC wherein the collagen/CMC ratio ranges from 3/1 to 1/3, preferably from 2/1 to 1/2, advantageously about 1/1 (w/w).

The term "collagen" denotes herein an animal collagen of any origin, e.g. from a mammal such as human, bovine or equine collagen, or from a bird, otherwise the collagen can be modified (e.g. it could be cross-linked) as well. Preferably the collagen is not cross-linked. The collagen, that can be used according to the invention, is highly purified and suitable for subcutaneous and dermal injections.

According to a preferred embodiment, the collagen is an equine collagen, advantageously native equine type I collagen.

According to a more preferred embodiment of the invention, the collagen is equine type I collagen in the form of a powder wherein at least 99.5% of the particles has maximum size of 80 microns and wherein at least 25% of the particles have particle size greater than 30 microns, preferably the amount of particles having size greater than 30 microns is 25-45%.

Unless otherwise indicated, the percentage of particles means herein "in volume" with respect to the total volume of the particles.

According to an embodiment of the invention, at least 15% of the particles have particle size greater than 40 microns; more preferably the amount of particles having size greater than 40 microns is 15% to 22%. Preferably, the amount of particles having particle size greater than 50 microns is 8 to 13%.

Still according to a preferred embodiment, the collagen powder does not contain more than 10% of particles having size of 10 microns or lower, and the amount of particles having size between 10 and 20 microns is 25% to 35%.

A preferred collagen is a combination of the above specified particle distributions, wherein 35% to 50% of the particles has size of 20-70 microns.

A preferred collagen according to the invention is the one described in WO2014/183770, in the name of the same applicant.

"CMC" or "carboxymethyl cellulose" means herein a carboxymethyl cellulose of any degree of substitution, preferably in the form of salt, more preferably as sodium salt. According to a preferred embodiment and opposite to what is currently used in the prior art, the CMC of the invention is advantageously a not cross-linked CMC.

The degree of substitution of a preferred CMC is 1.12 to 1.21 (highly substituted CMC).

The degree of substitution of another preferred CMC is 0.65 to 0.90 (poorly substituted CMC).

However other degrees of substitution can be selected.

Since a greater degree of substitution increases the filling in terms of steric hindrance and makes the molecule more slowly degradable, the field technician will be able to select the type of CMC to be used, depending on the intended use.

According to a preferred embodiment, the viscosity degree of CMC is 1500 to 3100 mPa (highly substituted CMC) or 200 to 800 mPa (poorly substituted CMC).

The analyses for the evaluation of the degree of substitution and viscosity have been carried out as recommended in the monograph of U.S. Pharmacopoeia (37th Edition; National Formulary n. 32).

The CMC that can be used according to the invention is commercially available; an example of a CMC suitable for the present invention is the one called "ERELLE™" currently sold by SWEDEN & MARTINA.

The use of a not cross-linked CMC has the advantage of better combining with collagen in the injectable suspension and moreover of being more easily extruded through the needle and injectable.

As mentioned above, in addition to the CMC also the collagen is preferably not cross-linked.

The combination of the invention is useful as dermal filler for the improvement of aesthetic defects and signs of aging, such as facial creases and depressions; to fill and level under eye and perioral wrinkles; to augment and/or remodel the lip volume; to correct nasolabial folds; to modify the form and volume of cheekbones, chin and forehead.

The combination is also useful to reduce or eliminate cicatricial results, depressions or furrows, originating for example from acne or surgeries.

The combination, for the use thereof, is advantageously held in a saline or isotonic solution, preferable buffered at pH of about 7.

The apyrogenic, sterile and injectable pharmaceutical compositions, comprising the combination of the invention held in a saline or isotonic solution, are a further subject-matter of the invention. Such compositions preferably comprise the combination in which the collagen/CMC ratio is 3/1 to 1/3 (w/w), preferably 2/1 to 1/2 (w/w), advantageously said ratio being 1/1 (w/w).

As mentioned, the compositions of the invention advantageously comprise a buffer system which maintains the composition at pH of about 7. pH about 7 means a physiologically compatible pH, which can vary for example between 6.8 and 7.5, e.g. 7.2 or 7.4.

A suitable buffer system is for example the phosphate buffer, usually used to buffer injectable solutions at pH of about 7.

The compositions of the invention can also comprise other active ingredients and/or additives, provided that they are pharmaceutically acceptable, suitable to be injected and compatible with the combination. Such further components can include, in addition to the buffer system, local anesthetics, such as lidocaine, stabilizers and/or preservatives.

The concentration of the combination in the compositions of the invention can vary from 0.5% to 3%, preferably around 2% and, as mentioned, according to a preferred embodiment the combination contained in the injectable compositions of the invention is constituted by collagen and CMC in 1/1 weight ratio.

The percentage amounts are conventionally expressed as "w/w", i.e. mg/mg. Therefore, a composition comprising 2% of the combination will contain 2 mg of the combination per each 100 mg of suspension.

According to a preferred embodiment, the compositions of the invention comprise about 1% of collagen and about 1% of CMC, i.e. 10 mg of collagen and 10 mg of CMC per 1 g of suspension.

Since the compositions of the invention have to be injected, they can be sterilized in compliance with known methods, e.g. but not exclusively, by gamma-ray exposure. The compositions of the invention, because of their subcutaneous or intradermal administration, are preferably packaged in media suitable for the use, advantageously in single-dose, graduated and ready to use syringes.

Such syringes are usually suitable to contain 1 to 5 ml of the composition and constitute a further subject-matter of the invention.

By way of example, the syringes of the invention comprise 0.5 ml to 5 ml of the composition of the invention, advantageously about 1 or 2 g of composition.

A preferred syringe contains 1 g of composition comprising 10 mg of collagen and 10 of CMC held in a saline or isotonic solution, buffered at pH of about 7. Subject-matter of the invention is also the combination and the composition according to the invention, for their use as dermal fillers.

Further subject-matter of the invention is a method for the improvement of aesthetic defects and signs of aging as defined above, or to reduce or eliminate the cicatricial results originating for example from acne or surgeries, comprising injecting a composition as defined according to the invention in the subcutis or dermis of a subject in need thereof.

The combination and compositions of the invention have the advantage of combining a filling effect with a physical mechanism of mechanic type (filler) mediated by CMC with a physiological tissue regenerative effect mediated by collagen.

In particular it has been observed that, thanks to the action of the CMC which occurs by collecting and keeping physiological liquids around the site of injection thanks to its tridimensional structure, the collagen injected at the same time is more quickly and uniformly dispersed together with the CMC inside the treated tissue, thus generating improved efficacy of the treatment with respect to products of the known art.

Moreover, it has been possible to observe that permanence times of the combination at the injection site are prolonged with respect to those of the two singly administered components. The result relative to the permanence times is particularly surprising considered that, in the preferred embodiment, both collagen and CMC are not cross-linked. Without wishing to provide a scientific explanation, a hypothesis is that an interaction between the COO- groups of the CMCNa and the NH₂ or -NH₃⁺ groups of the employed collagen is created and that this interaction is the reason of the observed technical effect.

Examples of the compositions are provided in the following Experimental section.

### Experimental Section

### Example 1

A disposable syringe containing the following composition is prepared:
- 10 mg of equine type I collagen
- 10 mg of not cross-linked CMCNa;
- Phosphate buffer
- Saline/buffered at pH 7 q.s. to 1 g

### Example 2

A disposable syringe containing the following composition is prepared:
- 10 mg of equine type I collagen, as defined in WO2014/183770
- 10 mg of not cross-linked CMCNa;
- Phosphate buffer
- Saline/buffered at pH 7 q.s. to 1 g

### Example 3

A disposable syringe containing the following composition is prepared:
- 20 mg of equine type I collagen
- 10 mg of not cross-linked CMCNa;
- Phosphate buffer
- Saline/buffered at pH 7 q.s. to 1 g

### Example 4

A disposable syringe containing the following composition is prepared:
- 10 mg of equine type I collagen
- 20 mg of not cross-linked CMCNa;
- Phosphate buffer
- Saline/buffered at pH 7 q.s. to 1 g

### Example 5

A disposable syringe containing the following composition is prepared:
- 10 mg of equine type I collagen
- 10 mg of not cross-linked CMCNa;
- 3 mg of lidocaine;
- Phosphate buffer
- Saline/buffered at pH 7 q.s. to 1 g

## Claims

1. Combination of collagen and carboxymethyl cellulose (CMC), for use as a dermal filler and as a tissue regenerating agent.

2. Combination according to claim 1, **characterized in that** it is a fixed composition wherein the collagen/CMC ratio is from 3/1 to 1/3 (w/w).

3. Combination according to claim 2, **characterized in that** it is a fixed composition wherein the collagen/CMC ratio is 1/1 (w/w).

4. Combination according to any one of claims 1 to 3, **characterized in that** said collagen is equine type I collagen, preferably not cross-linked.

5. Combination according to any one of claims 1 to 4, **characterized in that** said CMC is CMCNa and is not cross-linked.

6. Injectable composition, comprising the combination as defined in any one of claims 1 to 5.

7. Composition according to claim 6, **characterized in that** it comprises from 0.5 wt% to 3 wt% of said combination.

8. Composition according to claim 6 or 7, **characterized in that** it comprises 2 wt% of said combination.

9. Composition according to any one of claims 6 to 8, **characterized in that** said composition is held in a buffered saline or isotonic solution at about 7 pH.

10. Composition according to any one of claims 6 to 9, **characterized in that** it comprises 10 mg of collagen, 10 mg of CMC and a buffered saline or isotonic solution at about 7 pH q.s. to about 1 g.

11. Combination as defined in any one of claims 1 to 5 for use thereof as dermal filler and as a tissue regenerating agent.

12. Composition as defined in any one of claims 6 to 10 for use thereof as a dermal filler and as a tissue regenerating agent.

13. Pre-filled syringe containing a composition as defined in any one of claims 6 to 10.
